# EUROPÄISCHE PATENTSCHRIFT

(11) **EP 0 793 980 B1**
(45) Veröffentlichungstag und Bekanntmachung des Hinweises auf die Patenterteilung: **28.05.2003**
(21) Anmeldenummer: 97250055.7
(22) Anmeldetag: 04.03.1997
(51) Int. Cl.: A61N 1/372

(54) **Vorrichtung zur Speicherung von Signalen in einem implantierbaren medizinischen Gerät**
Signal storage device in an implantable medical apparatus
Dispositif de stockage de signaux dans un appareil medical implantable

(30) Priorität: 04.03.1996 DE 19609411
(43) Veröffentlichungstag der Anmeldung: 10.09.1997
(73) Patentinhaber: BIOTRONIK Mess- und Therapiegeräte GmbH & Co Ingenieurbüro Berlin, 12359 Berlin (DE)
(72) Erfinder: Wyborny, Paul, Lake Oswego, Oregon 97035 (US); Nigam, Indra, Lake Oswego, Oregon 97035 (US); Schaldach, Max, Prof. Dr.-Ing., 91054 Erlangen (DE)
(74) Vertreter: Christiansen, Henning

(56) Entgegenhaltungen:
- US-A- 5 312 446
- US-A- 5 333 615
- SHRIDHAR M ET AL: "ANALYSIS OF ECG DATA, FOR DATA COMPRESSION" INTERNATIONAL JOURNAL OF BIO-MEDICAL COMPUTING, Bd. 10, 1. Januar 1979, Seiten 113-128, XP000568228
- BLANCHARD S.M. ET AL: 'Comparison of methods for adaptive sampling of cardiac electrograms and electrocardiograms' MEDICAL & BIOLOGICAL ENGINEERING & COMPUTING Bd. 23, Juli 1985, Seiten 377 - 386

## Beschreibung

Die Erfindung betrifft ein Verfahren zur Speicherung und/oder Übertragung von einen zeitlichen Verlauf auf weisenden Signalen sowie eine Vorrichtung zur Durchführung des Verfahrens.

In engem Zusammenhang mit dem ständig weitere Verbreitung erlangenden Einsatz implantierbarer medizinischer Geräte, wie etwa Herzschrittmachern oder Defibrillatoren, steht die Erfassung und Auswertung von physiologisch relevanten Signalen im Körper durch geeignete Erfassungs- bzw. Überwachungseinrichtungen. Auf der Grundlage der Ergebnisse - beispielsweise der Erfassung und Auswertung des zeitlichen Verlaufes von Herzaktionspotentialen oder des Herzinnendrucks bzw. volumetrischer Impedanzmessungen etc. - wird der Betrieb des implantierbaren Gerätes gesteuert. Zusätzlich oder auch unabhängig von der erstgenannten Funktion können die Daten für eine separate ärztliche Diagnose und/oder eine Betriebszustandsanalyse des Gerätes selbst genutzt werden.

Die relevanten Signale fallen in der Regel in analoger Form als Meßsignale an und müssen für viele Anwendungen über einen relativ langen Zeitraum mit hoher Auflösung registriert und für eine Auswertung bereitgehalten werden. Dies erfordert bei konventioneller Handhabung die Speicherung und/oder Übertragung einer großen Datenmenge im implantierten Gerät bzw. nach außerhalb und letztlich deren angemessene Verarbeitung. Die rasanten Fortschritte bei der Herstellung kostengünstiger Halbleiterspeicher mit sehr großen Speicherkapazitäten und in der Prozessortechnik haben die diesbezüglichen Möglichkeiten bei implantierbaren Geräten wesentlich vergrößert; dennoch ist zur Kostensenkung und zu einem möglichst stromsparenden Betrieb eine Beschränkung der Datenmenge wünschenswert. Dies ist besonders aktuell für Geräte, bei denen die Erfassung und Handhabung mehrerer Arten relevanter Daten über Zeiträume von Tagen und Wochen wünschenswert ist, beispielsweise für automatische Herzschrittmacher/Kardioverter.

Ein aus einer Vielzahl von technischen Anwendungen - insbesondere der Kommunikations- und Meßtechnik - bekannter Ausweg besteht in der Datenkompression. Hierfür gibt es verschiedene Verfahren, die in der einschlägigen Literatur ausführlich beschrieben sind.

Auf dem Gebiet der Speicherung ud Übertragung von physiologischen Daten, speziell auch bei implantierbaren Geräten, haben sich diese Techniken bislang nicht durchsetzen können - teils wegen des hohen gerätetechnischen Aufwandes, teils auch im Hinblick auf die Spezifik der hier auftretenden Signale und der im Zuge ihrer Verarbeitung zu gewinnenden Informationen. Daher werden auf diesem Gebiet üblicherweise nach wie vor die anfallenden Daten ohne Verdichtung in digitalisierter Form gespeichert.

Dieser Weg wird auch beim EKG-Monitor nach der EP-0 512 667-A1 beschritten, der als Speichermedium eine Diskette bzw. einen ähnlichen Datenträger nutzt.

In der europäischen Patentschrift EP-0 149 094-B1 ist ein nicht implantierbares Aufzeichnungsssystem für die zeitkomprimierte Registrierung von EKG-Wellenformen beschrieben, dessen Kern einfach in der Verringerung des Auflösungsvermögens einer konventionellen Registrierung auf Aufzeichnunngspapier besteht, die hier mittels eines Thermo-Zeilendruckers erfolgt. Das System dient nur einem ersten Screening im Vorfeld der späteren genaueren Erfassung von Arrythmiezuständen und ist für die Gewinnung genauer diagnostischer Aussagen sowie speziell für die Steuerung implantierter Geräte grundsätzlich ungeeignet.

In der europäischen Patentanmeldung EP-0°263°599-A2 ist eine Vorrichtung zur EKG-Datenkompression zwecks Speicherung von EKG-Daten in einem Herzschrittmacher beschrieben. Diese geht davon aus, daß während des größten Teils eines Herzzyklus das EKG-Signal konstant ist (nahe der Nullinie liegt), so daß für diese Zeiten kein Signalwert gespeichert werden muß. Gespeichert wird daher die Zeitspanne, die jeweils zwischen vorgegebenen Änderungen des EKG-Signals verstreicht, womit das Verfahren eine auf die Zeit bezogene Variante der bekannten Deltamodulation darstellt. Wendepunkte im Signalverlauf werden durch zusätzliche Speicherung einer Flag gekennzeichnet.

Soll damit eine Auflösung realisiert werden, die die zuverlässige autonome Steuerung eines kombinierten Antiarrhythmiegerätes erlaubt, wird sehr viel Speicherplatz für die Speicherung der genauen Zeitpunkte der Signaländerungen benötigt, und die Aufbereitung der Zeitdaten für die eigentliche (insbesondere vergleichende) Auswertung erfordert erheblichen Aufwand.

In der US-Patentschrift Nr. 5 312 446 ist ein implantierbares medizinisches Gerät mit Telemetrieeinrichtung beschrieben, bei dem eine Speicherung erfaßter physiologischer Signale unter - wahlweise mehrstufiger - Datenkompression vorgesehen ist. Der Kompressionsvorgang beruht hier auf der schrittweisen Aussonderung weniger signifikanter Meßpunkte aufgrund eines Vergleichs der Absolut-Meßwerte an aufeinanderfolgenden Abtastpunkten. Die Speicherung der (komprimierten) Daten erfolgt in analoger Form, was das Vorsehen eines speziellen Speichers voraussetzt.

In S.M. Blanchard, R.C. Barr: "Comparison of methods for adaptive sampling of cardiac electrograms and electrocardiograms", Medical & Biological Engineering & Computing, July 1985, 377, werden verschiedene Verfahren auf ihre Eignung zur Datenkompression von EKG-Signalen hin verglichen, darunter sind auch Verfahren beschrieben, welche die Änderung der Steigung des Signalverlaufs auswerten.

Ein Verfahren gemäß dem Oberbegriff von Anspruch 1 ist in SHRIDHAR M. et al. "ANALYSIS OF ECG DATA, FOR DATA COMPRESSION", INTERNATIONAL JOURNAL OF BIO-MEDICAL COMPUTING, Bd. 10, 1. Januar 1979, Seiten 113 - 128 beschrieben.

Der Erfindung liegt die Aufgabe zugrunde, dieses Verfahren derart weiterzubilden, dass der Speicherbedarf weiter verringert werden kann. Außerdem ist es eine Aufgabe der Erfindung, eine Vorrichtung zur Durchführung des Verfahrens zur Verfügung zu stellen.

Diese Aufgabe wird durch ein Verfahren mit den Merkmalen des Anspruchs 1 sowie eine Vorrichtung nach Anspruch 7 oder Anspruch 8 gelöst.

Die Erfindung schließt den Gedanken ein, diejenigen Abschnitte eines physiologischen (oder allgemein durch seinen zeitlichen Verlauf gekennzeichneten) Signals zur Speicherung bzw. Übertragung auszuwählen, in denen der zeitliche Verlauf sich wesentlich ändert, während eine Speicherung bzw. Übertragung von Signalabschnitten, in denen keine wesentliche Änderung stattfindet, vermieden wird.

Der Verlauf in den letzteren Abschnitten kann dann aufgrund der gespeicherten Signalproben mit ihrem zeitlichem Bezugspunkt approximiert werden, womit insgesamt eine hinreichend genaue Rekonstruktion des gesamten Signals unter Einsparung von Speicher- bzw. Übertragungskapazität möglich wird. Die Einsparung kann speziell bei Signalen in der Art von EKG-Signalen, bei denen lange Abschnitte mit nur geringfügigen Amplitudenänderungen und kurze Abschnitte nebeneinander existieren, in denen schnelle und für die Auswertung entscheidende Änderungen stattfinden, ganz erheblich sein.

Die erfindungsgemäße Vorrichtung ist gleichermaßen zur Kompression von aktuell im oder am Körper eines Patienten aufgenommenen Signalen wie von aus anderen Quellen stammenden Modell- bzw. Vergleichsdaten geeignet, die im medizinischen Gerät zu dessen Steuerung oder zu Diagnosezwecken benötigt werden. Von besonderem Nutzen ist die Anwendung bei der Speicherung bzw. Übertragung von Kurz- und Langzeit-EKGs oder Histogrammen zur Herztätigkeit, speziell von im Rahmen von Tagesprofilen oder Belastungszyklen oder bei vorliegenden Tachykardien aufgenommenen Daten bzw. entsprechenden Modelldaten.

Die erfindungsgemäße Lösung ermöglicht in diesen Zusammenhängen eine erhebliche Erhöhung der Steuerungs-Intelligenz implantierter Schrittmacher, Defibrillatoren, Kardioverter oder Medikamentendosiergeräte, basierend auf wesentlich erweiterten Möglichkeiten zu einer echten Signalformverarbeitung zusätzlich zur üblichen Verarbeitung singulärer logischer oder skalarer Meß- und Vergleichsgrößen.

In vorteilhafter Weiterbildung der Vorrichtung wird für jede gespeicherte und/oder übertragene Signalprobe das Vorzeichen, der Amplitudenwert und der Zählwert der gegenüber der zuletzt gespeicherten Signalprobe verstrichenen Abtastintervalle in einem Datenwort gespeichert bzw. übertragen. Dessen Wortbreite richtet sich nach der Verarbeitungsbreite der eingesetzten Hardwarekonfiguration.

Die Vorrichtung ermöglicht eine zeitnahe Übertragung der erfaßten und komprimierten Daten ohne kostenaufwendige Pufferspeicherung umfangreicher Datensätze. Da es einen Rückgriff auf die letzte als speicherungs- bzw. übertragungswürdig bewertete Signalprobe erfordert, ist jedoch im Falle der zeitnahen Übertragung mindestens die jeweils zuletzt übertragene Signalprobe intern zwischenzuspeichern.

Die mit der erfindungsgemäßen Vorrichtung komprimierten Signale können vor allem Herzaktionssignale, insbesondere ein intrakardial aufgenommenes EKG, oder solche darstellende, von außerhalb des Körpers des Patienten übermittelte, Modell- bzw. Vergleichssignale sein. Es ist aber auch auf andere zeitabhängige physiologische Signale oder Vergleichssignale anwendbar.

Die Vorrichtung läßt sich besonders einfach und kostengünstig in der Hardware-Umgebung moderner Defibrillatoren und Schrittmacher realisieren, wenn bereits die Abtastung mit einer Digitalisierung der Amplitudenwerte verbunden ist, wobei es von besonderem Vorteil für die adäquate Speicherung und weitere Auswertung speziell von Herzsignalen ist, wenn die Digitalisierung kleiner Amplitudenwerte mit höherer Auflösung erfolgt als die größerer Amplitudenwerte.

Der Signalaufnehmer wird hierbei mindestens eine - in der Regel intrakardiale, gegebenenfalls. aber auch epikardial angeordnete - Elektrode mit nachgeordnetem Meßverstärker zur Erfassung von Herzaktionspotentialen sein.

Bei der Vorrichtung gemäß der Erfindung wird der Speicher für die komprimierten Signale insbesondere als Halbleiter-Direktzugriffsspeicher (Schreib-Lese-Speicher, RAM) ausgebildet sein und eine Mehrzahl von getrennt zugreifbaren Speicherbereichen zur Speicherung jeweils einer Signalproben-Teilfolge, d.h. eines komprimierten Gesamt-Signals, aufweisen. Dies ermöglicht den unmittelbaren Vergleich der zu verschiedenen Zeitpunkten aufgenommenen Signale miteinander und/oder mit Vergleichssignalen.

In der eine Übertragung nach außen vorsehenden Ausbildung ist die Sendeeinheit als (an sich bekannte) Telemetrie-Sendeeinheit ausgestaltet.

In für die Mehrzahl der praktischen Anwendungen vorteilhafter Weise kann sowohl ein interner Speicher als auch eine Sendeeinheit vorgesehen sein, wobei die Sendeeinheit mit dem Speicher zur Übertragung von Speicherinhalten nach außerhalb des Gerätes verbindbar ist und (interne oder externe) Steuermittel eine Aktivierung der Sendeeinheit auf Abruf oder in vorgegebenen Zeitabständen bewirken. Dies ermöglicht beispielsweise einerseits die autonome, zeitnahe Aktivierung eines Defibrillators oder Kardioverters im (nahezu sofortigen) Ansprechen auf die Erfassung von Signalformen, deren Vergleich mit früher aufgenommenen Signalen oder Modellsignalen einen Therapiebedarf signalisiert. Andererseits werden damit diagnostisch wertvolle Primärund Sekundärdaten (Signalform-Vergleichsdaten) dem Arzt für die genauere Interpretation zugänglich gemacht.

Eine weitgehend interne Auswertung der komprimiert gespeicherten Signalformen bis hin zu Resultaten von unmittelbar diagnostischer Aussagekraft ist in vorteilhafter Weise möglich, wenn eine Steuer- oder Diagnosedaten-Berechnungseinheit vorgesehen ist, die eingangsseitig mit dem Speicher für die Signalproben-Teilfolgen und ausgangsseitig wahlweise mit einem Steuer- oder Diagnosedatenspeicher verbunden und zur vergleichenden Verarbeitung verschiedener gespeicherter Signalproben zur Gewinnung von Steuersignalen oder diagnostisch relevanten Daten ausgebildet ist.

Von besonderer praktischer Bedeutung ist die Einbeziehung der Vorrichtung in ein implantierbares Antiarrhythmiegerät zur Behandlung von Arrythmien des Herzens, insbesondere einen - oben bereits angesprochenen - Defibrillator und/oder Herzschrittmacher oder ein Medikamentendosiergerät, wobei Bauteile oder -gruppen dieser Geräte zur Implementierung der Vorrichtung mitbenutzt werden können.

Bei einem derartigen implantierbaren Antiarrhythmiegerät ist eine eingangsseitig mit dem Ausgang der oben erwähnten Steuerdaten-Berechnungseinheit und/oder dem Steuerdatenspeicher in Verbindung stehende Betriebssteuereinheit vorgesehen, welche zur Steuerung des Betriebs des Gerätes aufgrund des Ergebnisses der vergleichenden Auswertung ausgebildet ist. Dies wird in aller Regel eine Mikroprozessorsteuerung sein, die nach einem entsprechenden Betriebsprogramm arbeitet.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Fig. 1 eine schematische Darstellung eines Teils eines EKG-Signales zusammen mit dessen Repräsentation gemäß einem Ausführungsbeispiel der Erfindung,
Fig. 2 ein Flußdiagramm zum Ausführungsbeispiel gemäß Figur 1,
Fig. 3 eine schematische Darstellung der Datenwortstruktur bei der zuvor dargestellten Ausführungsform der Erfindung,
Fig. 4 eine Kodierungstabelle für die Signalamplituden die unter anderem mit der in den Figuren 1 bis 3 dargestellten Ausführungsform der Erfindung zur Anwendung gelangen kann,
Fig. 5 ein Zeitdiagramm zu der Variante gemäß Figur 4,
Fig. 6 ein Blockschalt- und Signallaufbild einer achaltungsmäßig ausgestalteten Ausführungsform der erfindungsgemäßen Vorrichtung,
Fig. 7 ein zu Fig. 6 gehöriges Schema der Steuersignalzustände und
Fig. 8 ein vereinfachtes Funktions-Blockschaltbild eines Herzstimulators als Ausführungsform der Erfindung.

Die nachfolgende Beschreibung eines Ausführungsbeispiels der Erfindung erfolgt in funktioneller Form, wobei die Realisierung als Vorrichtung - wie weiter unten beschrieben - sowohl in logisch diskreter als auch in programmierter Form erfolgen kann. Die Umsetzung erfolgt dann beispielsweise entweder in ein entsprechendes Logik-Array oder in ein Steuerprogramm, das in ein EPROM eingelesen wird.

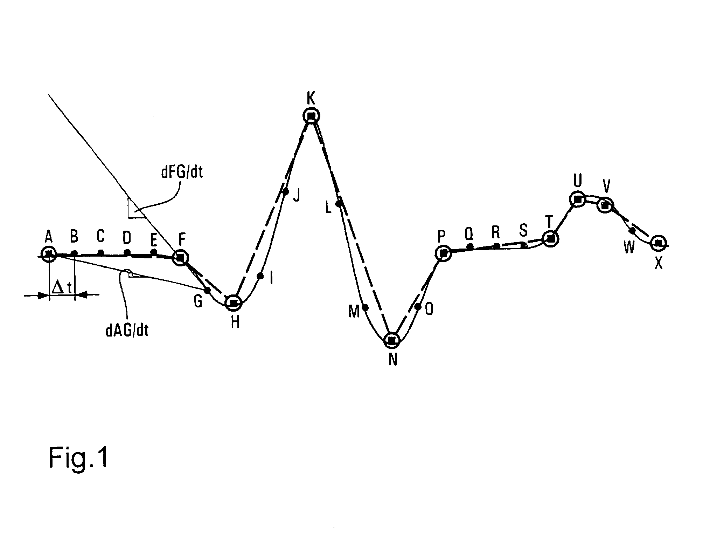

In der Darstellung gemäß Fig. 1 ist zur Verdeutlichung des Grundgedankens der Erfindung in vereinfachter Darstellung ein QRS-Komplex als Teil eines EKG-Signales (durchgezogene Linie) zusammen mit dessen Approximation (gestrichelte Linie) wiedergegeben. Die fortlaufend mit Großbuchstaben bezeichneten gefüllten Kreise und Quadrate stellen die mit konstantem Abtastintervall At gewonnenen Signalproben dar; die Quadrate kennzeichnen dabei die im Ergebnis gespeicherten bzw. übertragenen Signalproben.

Am Beispiel der Signalproben G als aktuelle und F als vorhergehende Signalprobe wird das Prinzip der Erfindung verdeutlicht: Der Anstieg dFG/dt der geraden Verbindungslinie zwischen den Punkten F und G wird bestimmt und mit dem Anstieg dAG/dt der geraden Verbindungslinie zwischen dem Punkt G und dem Punkt A, der letzten gespeicherten Signalprobe, verglichen. Da die Differenz (dFG/dt - dAG/dt) einen vorbestimmten - in der Grafik nicht gezeigten - Wert übersteigt, wird die Signalprobe F als nunmehr letzte unter Angabe des Vorzeichens, des Amplitudenwertes und des Zählwertes der seit der letzten Speicherung (oder seit einem definierten Zeitpunkt O) verstrichenen Abtastintervalle gespeichert.

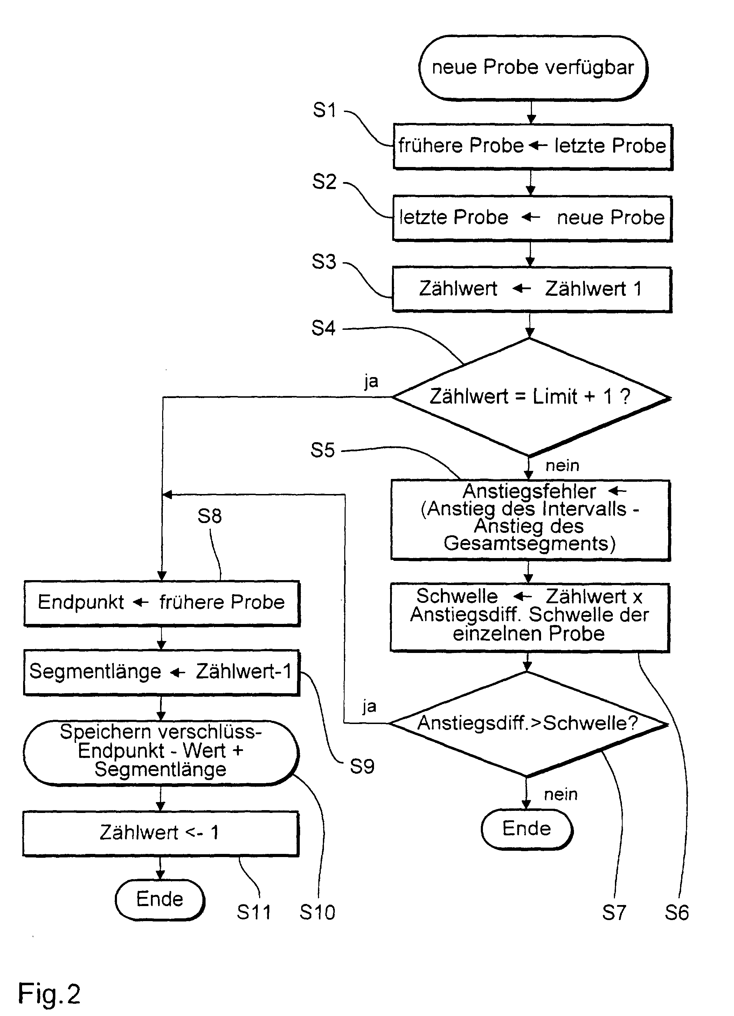

Die Signalprobe F ist somit den weiteren Vergleichen zugrundezulegen. Da bei den Signalproben D und E das gleiche Vorgehen jeweils eine unterhalb des Schwellwertes liegende Differenz der Anstiegswerte ergeben hat, wurden diese Signalproben nicht gespeichert. (Das Vorgehen für die Signalproben A bis C wird aus dem dargestellten Signalabschnitt allein nicht ersichtlich; hierfür wird auf zeitlich vor "A" liegende Signalabschnitte oder spezielle Startbedingungen für die Prozedur zurückgegriffen, die hier jedoch nicht getrennt erörtert werden.) Fig. 2 illustriert anhand eines Flußdiagramms den Ablauf des erfindungsgemäßen verfahrens anhand einer Ausführungsform, bei der der Startpunkt durch das Vorliegens einer (beliebigen) neuen Signalprobe gebildet wird:

In einem ersten Schritt S1 wird die bis zur Bereitstellung der neuen Signalprobe aktuelle Signalprobe als "frühere Probe" umdefiniert, in einem Schritt S2 wird die neu gewonnene Signalprobe als "aktuelle Probe" an die Stelle der ersteren gesetzt, in einem Schritt S3 wird der Zählwert um eins inkrementiert, und in einem Schritt S4 wird entschieden, ob der neue Zählwert um eins über einem vorgegebenen Limit liegt. Hier verzweigt das Flußdiagramm:

Ist dies nicht der Fall ("Nein"), wird zu einem Schritt S5 weitergegangen, in dem der Betrag der Differenz aus dem Anstieg im letzten Meßintervall und dem Anstieg des Gesamtsegments zwischen der aktuellen Probe und der letzten gespeicherten Probe bestimmt und als Anstiegsdifferenz definiert wird. In einem nachfolgenden Schritt S6 wird das Produkt aus dem aktuellen Zählwert und einem für ein Abtast-Zeitintervall festgelegten Schwellwert einer zulässigen Abweichung der Anstiegs-Differenzwerte gebildet und als aktueller Schwellwert definiert, der - wie oben erläutert - einem Vergleich der Anstiege im letzten Meßintervall und im Gesamtintervall bis zurück zur letzten gespeicherten Probe zugrundezulegen ist. Im folgenden Schritt S7 wird bestimmt, ob die im Schritt S5 bestimmte Anstiegsdifferenz größer als der im Schritt S6 bestimmte Schwellwert ist.

Hier verzweigt sich das Flußdiagramm abermals: Ist die Anstiegsdifferenz nicht größer als der Schwellwert ("Nein"), endet die Schrittfolge der Entscheidungsprozedur, ob die frühere Signalprobe zu speichern bzw. zu übertragen ist, damit, daß keine Speicherung oder Übertragung initiiert wird.

Ist sie jedoch größer ("Ja"), mündet der Ablauf in den Pfad, der beim Schritt S4 mit dem Fall begonnen wurde, daß der neue Zählwert um eins über dem Limit liegt ("Ja" beim Schritt S4). Im ersten Schritt dieses Pfades, S8, wird die frühere Probe als neuer Endpunkt definiert. In einem nächsten Schritt S9 wird mit dem um eins dekrementierten aktuellen Zählwert die Segmentlänge definiert. In einem nachfolgenden Schritt S10 werden der im Schritt S8 definierte Endpunkt (genauer gesagt: dessen Vorzeichen und Amplitudenwert in kodierter Form - siehe hierzu weiter unten) sowie die im Schritt S9 definierte Segmentlänge gespeichert, und schließlich wird im Schritt S11 "1" als neuer Zählwert gesetzt, woraufhin das Programm in diesem Pfad endet.

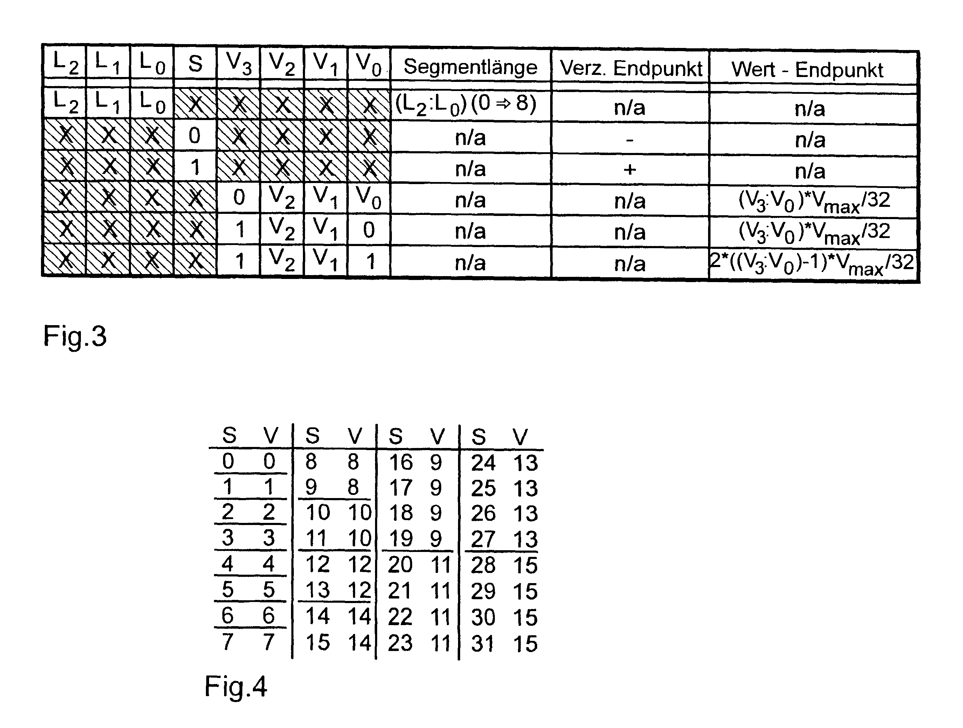

Fig. 3 gibt in einer schematischen Darstellung ein Beispiel für die Datenwortstruktur an, die bei einer bevorzugten Ausführung der Speicherung der Signalproben im Rahmen der Vorrichtung benutzt wird. Es gibt - wie in der Figur zu erkennen - Datenwortfelder für die zeitliche Zuordnung der Signalprobe (Segmentlänge - L₂ bis L₀), für das Vorzeichen der Signalamplitude (Endpunkt-Vorzeichen s) und den Amplitudenwert (Endpunkt-Wert - V₃ bis V₀). Die in der Figur gezeigte spezielle Struktur im letztgenannten Feld eignet sich zu einer effizienten Ausnutzung der verfügbaren Wortbreite - hier 8 bit - zur Speicherung kleiner Signalamplituden mit gegenüber großen Amplituden höherer Auflösung. Zu diesem Zweck ist die Signalamplitude über die Kombination von V₃ und V₀ (vgl. die letzte Spalte der Tabelle) einer von drei Genauigkeitszonen zugeordnet.

Hinsichtlich des Segmentlängen-Feldes ist anzumerken, daß ein kodierter Wert von "0" für eine Segmentlänge mit dem Wert "8" steht, da eine Segmentlänge Null nicht auftritt. Vₘₐₓ steht in der Figur für die maximale Größe der (digitalisierten) Signalproben-Amplitude und ergibt sich als Vₘₐₓ = 2^{(#bits 1)}, worin "#bits" die Verarbeitungsbreite bei der A/D-Wandlung ist. Eine größere Wortbreite ermöglicht natürlich - gleichbleibende Maximalamplitude vorausgesetzt - wegen der größeren verfügbaren Länge für das S-und V-Feld eine höhere zeitliche und/oder Amplitudenauflösung.

In der Darstellung gemäß Fig. 4 ist ein Beispiel für eine Kodierungstabelle für ein Signal mit Signalamplitudenwerten S von 0 bis 31 bei 6-Bit-Verschlüsselung (1 Bit für das Vorzeichen, 5 Bit für den Amplitudenwert) in kodierte Amplitudenwerte V wiedergegeben. Die unterstrichenen Werte bezeichnen jeweils das Erreichen einer neuen Stufe im kodierten Wert. Die vergleichsweise höhere Auflösung bei niedrigen S-Werten ist gut erkennbar.

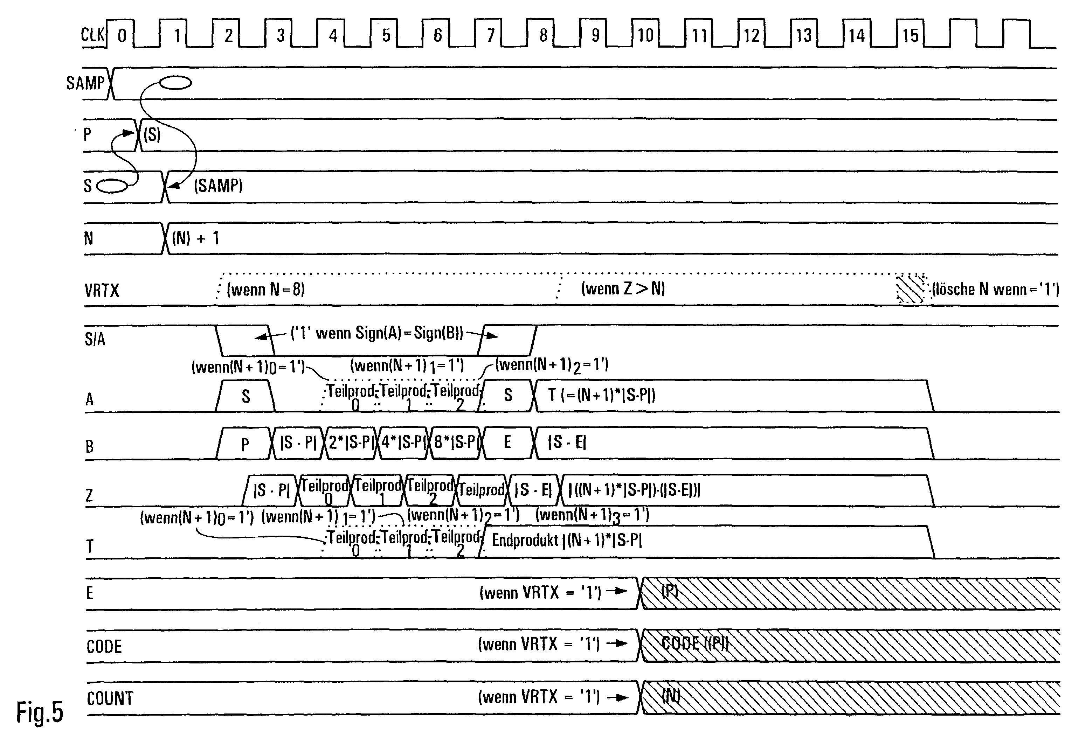

Bei der in Fig. 5 gezeigten schematischen Darstellung eines Zeitdiagramms zu einem Ausführungsbeispiel (entsprechend Fig. 2) des Steuerungsablaufs gemäß der Erfindung sind folgende Abkürzungen verwendet:
- CLK: = Takt
- SAMP: = neues EKG-Signal im Format Vorzeichen/Wert
- P: = früherer Signalproben-Wert
- S: = aktueller Signalproben-Wert
- N: = Segmentlängenzähler
- VRTX: = Steuersignal, das gesetzt wird, wenn der Segment-Endpunkt angefordert wird.
- S/A: = ALU-Betriebsartsteuersignal
- A: = ALU-Eingaberegister
- B: = ALU-Eingaberegister
- Z: = ALU-Ausgaberegister
- T: = Zwischenspeicher-Register
- E: = Segment-Endpunktwert
- CODE: = kodierter Segment-Endpunkt
- COUNT: = Segmentlängenwert.

Das Setzen des Steuersignals "VRTX" erfolgt dabei, wenn die maximal zulässige Segmentlänge - in der Beschreibung zu Fig. 2 auch als "Limit" bezeichnet erreicht ist oder die festgestellte Anstiegsänderung die Definition eines neuen Endpunktes erfordert.

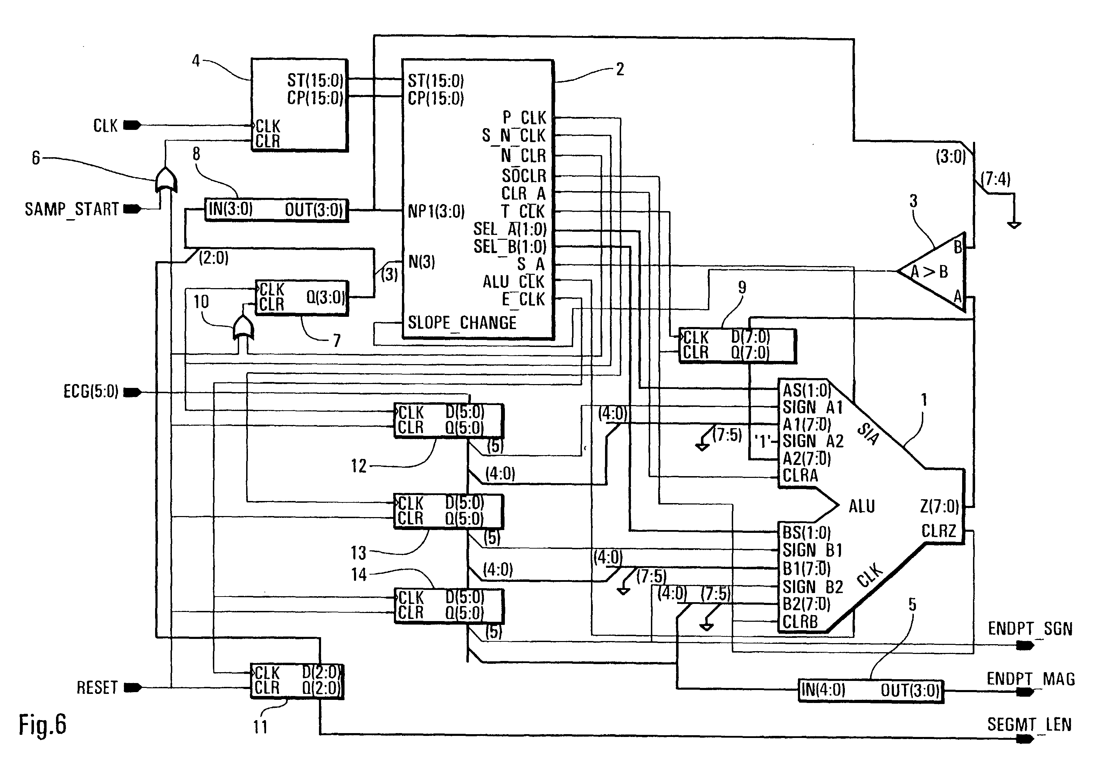

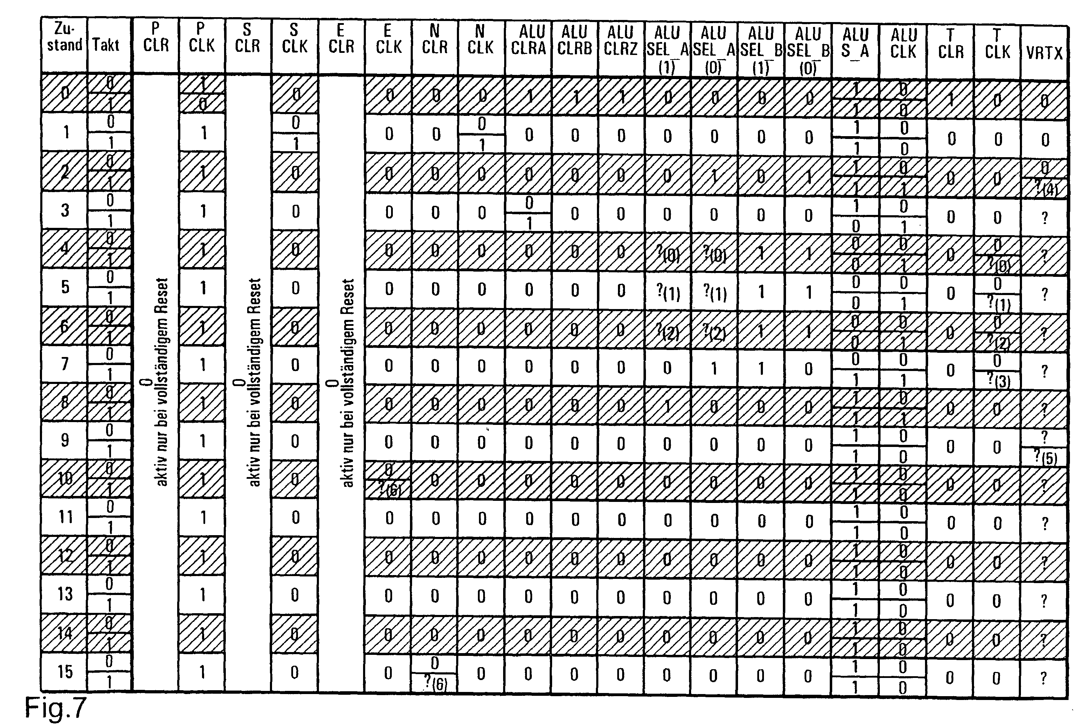

In der nachfolgenden Beschreibung wird auf die Figuren 6 und 7 Bezug genommen, wobei Fig. 6 ein Blockschalt- und Signallaufbild einer Ausführungsform der erfindungsgemäßen Vorrichtung, die den in Fig. 2 und 5 skizzierten Ablauf realisiert, und Fig. 7 ein dazugehöriges Schema der Steuersignalzustände wiedergibt.

Der Vorrichtung werden - wie am linken Rand von Fig. 6 dargestellt - von in der Figur nicht gezeigten Einrichtungen die Eingangssignale "CLK" (Taktsignal, von einem Taktgeber), "SAMP_START" (Startsignal, beispielsweise von einer Eingangsstufe mit zusätzlichem Steuersignalausgang), "ECG[5:0]" (Signalprobe, vom A/D-Wandler als 5-Bit-Bussignal) und "RESET" zugeführt. Sie gibt - wie am rechten Rand der Figur zu erkennen - im Ergebnis der Verarbeitung die Bus-Datensignale "ENDPT_SGN" (Vorzeichen der Signalamplitude), "ENDPT_MAG" ([Absolut-] Wert der Signalamplitude) und "SEGMT_LEN" (Segmentlänge, die zeitliche Zuordnung der Probe definierend) zum Zwecke der internen Speicherung oder Übertragung zu einem externen Gerät - nach Bildung eines einzelnen Datenwortes hieraus - aus.

Zur Spezifizierung der in der Figur angegebenen internen Signale wird auf die Zustandstabelle gemäß Fig. 7 verwiesen, eine verbale Beschreibung der Vorrichtung und des Zusammenwirkens der Komponenten sowie der Signalflüsse wird daher nachfolgend lediglich in knapper Form gegeben.

Hauptkomponenten der gezeigten Vorrichtung sind eine Berechnungs- und Logikeinheit (ALU) 1 und ein Steuerdekoder 2, der ausgangsseitig mit verschiedenen Signal- und Steuereingängen der ALU verknüpft ist und deren Betrieb taktet, sowie ein Komparator 3. Diesen Komponenten sind auf die in der Figur gezeigte Weise ein Zustandszähler 4, ein Amplitudenwert-Kodierer 5 und mehrere Logikgatter und Register zugeordnet. Das externe Taktsignal wird direkt dem Zustandszähler 4 zugeführt, und das Signal "SAMP_START" wird über ein eingangsseitig des Zustandszählers 4 vorgesehenes ODER-Gatter 6, an dessen zweiten Eingang das Signal "RESET" gelangt, dem Rücksetzeingang des Zustandszählers 4 zugeführt.

Der Zustandszähler 4 ist ausgangsseitig mit dem Steuerdekoder 2 verbunden. Ebenfalls ausgangsseitig mit dem Steuerdekoder verbunden sind ein erstes Segmentlängenregister (Zähler) 7 und ein - seinerseits eingangsseitig mit letzterem verbundenes - zweites Segmentlängenregister (Inkrementierer) 8 und schließlich der Signalanstiegs-Komparator 3, der seinerseits über seinen einen Eingang vom Ausgangssignal des zweiten Segmentlängenregisters 8 und über seinen zweiten Eingang von der ALU 1 und einem T-Register 9 - siehe weiter unten gespeist wird. Mit dem Rücksetzeingang des ersten Segmentlängenregisters ist ein zweites ODER-Gatter 10 verbunden, an dessen einem Eingang das Signal "RESET" und an dessen anderem Eingang ein vom Steuerdekoder 2 erzeugtes Rücksetzsignal "N_ CLR" anliegt. Am Takteingang des ersten Segmentlängenregisters 7 liegt das vom Steuerdekoder 2 erzeugte Taktsignal "N_P_CLK" an.

Das Ausgangssignal des ersten Segmentlängenregisters bzw. Segmentlängenzählers 7 wird auch einem weiteren (dritten) Segmentlängenregister 11 zugeführt, dessen Takteingang vom Steuerdekoder 2 mit dem Taktsignal "E_CLK" versorgt wird, dessen Rücksetzeingang das Signal "RESET" empfängt und an dessen Ausgang als Ausgangssignal der Vorrichtung "SEGMT_ LEN" bereitsteht.

Das aktuelle Signalproben-Eingangssignal "ECG[5:0]" wird dem Signaleingang eines S-Registers (Register für den aktuellen Signalproben-Amplitudenwert) 12 zugeführt, an dessen Takteingang das vom Steuerdekoder 2 erzeugte Taktsignal "S_ N CLK" und an dessen Rücksetzeingang "RESET" anliegt. Der Ausgang des S-Registers 12 ist mit dem Signaleingang eines P-Registers 13 (Register für den früheren Signalproben-Amplitudenwert) verbunden, und es liefert weiterhin (getrennt) ein Vorzeichen-Ausgangssignal und ein Amplitudenwert-Ausgangssignal an die ALU 1. Das P-Register 13 ist analog beschaltet wie das S-Register und ausgangsseitig mit einem weiteren Register, dem E-Register 14 (für den Segment-Endpunktwert) verbunden, welches eingangsseitig wiederum analog beschaltet ist und als Ausgangssignale "ENDPT_SGN" und ein (unkodiertes) Amplitudensignal liefert. Das letztere wird einem Eingang der ALU 1 sowie dem Eingang des Kodierers 5 zugeführt, der seinerseits das kodierte Ausgangssignal "ENDPT_MAG" liefert.

In der Vorrichtung ist schließlich das T-Register 9 als Zwischenspeicher vorgesehen, dessen Signaleingang mit dem Signalausgang der ALU 1 verbunden ist, dessen Betrieb wiederum vom Steuerdekoder 2 getaktet wird und dessen Rücksetzeingang mit je einem Löschsignalausgang des Steuerdekoders 2 und der ALU 1 verbunden ist.

Der Betrieb der Vorrichtung ergibt sich (unter Beachtung der Signalzustandstabelle der Fig. 7) aus dem Flußdiagramm von Fig. 2 und dem Timingdiagramm von Fig. 5 und wird daher hier nicht nochmals näher beschrieben.

Für Fig. 7 gelten folgende Anmerkungen:
?₍₀₎ Steuerwert = '1' genau dann, wenn [N+1]0 = '1' (d.h. LSB des Ergebnisses der Inkrementierung des Zählwertes um eins)
?₍₁₎ Steuerwert = '1' genau dann, wenn [N+1]₁ = '1'
?₍₂₎ Steuerwert = '1' genau dann, wenn [N+1]₂ = '1'
?₍₃₎ Steuerwert = '1' genau dann, wenn [N+1]₃ = '1'
?₍₄₎ Steuerwert = '1' genau dann, wenn [N] = 8, d.h. Segment-Zählwert maximal)
?₍₅₎ Steuerwert = '1' genau dann, wenn [Z] > [N] d.h Anstiegsänderung größer als 1*LSB pro Abtastintervall)
?₍₆₎ Steuerwert = '1' genau dann, wenn VRTX = '1'

Die Steuersignale "ALU S_A" und "VRTX" müssen - etwa in einem SR-Flipflop zwischengespeichert ("gelatcht") werden, damit ihre jeweiligen Zustände während der Zustandszählwertänderungen stabil bleiben.

Die Darstellung der Fig. 6 zeigt eine Vorrichtung mit einem Eingangskanal, kann jedoch auf zwei oder mehrere Kanäle erweitert werden, wobei dann gegebenenfalls. eine Synchronisation der Datenflüsse in der Art vorgesehen sein kann, daß jedesmal dann, wenn die Anstiegsänderung des Signals in einem der Kanäle die Definition eines neuen Endpunktes erfordert, gleichzeitig ein solcher auch in den übrigen Kanälen definiert wird.

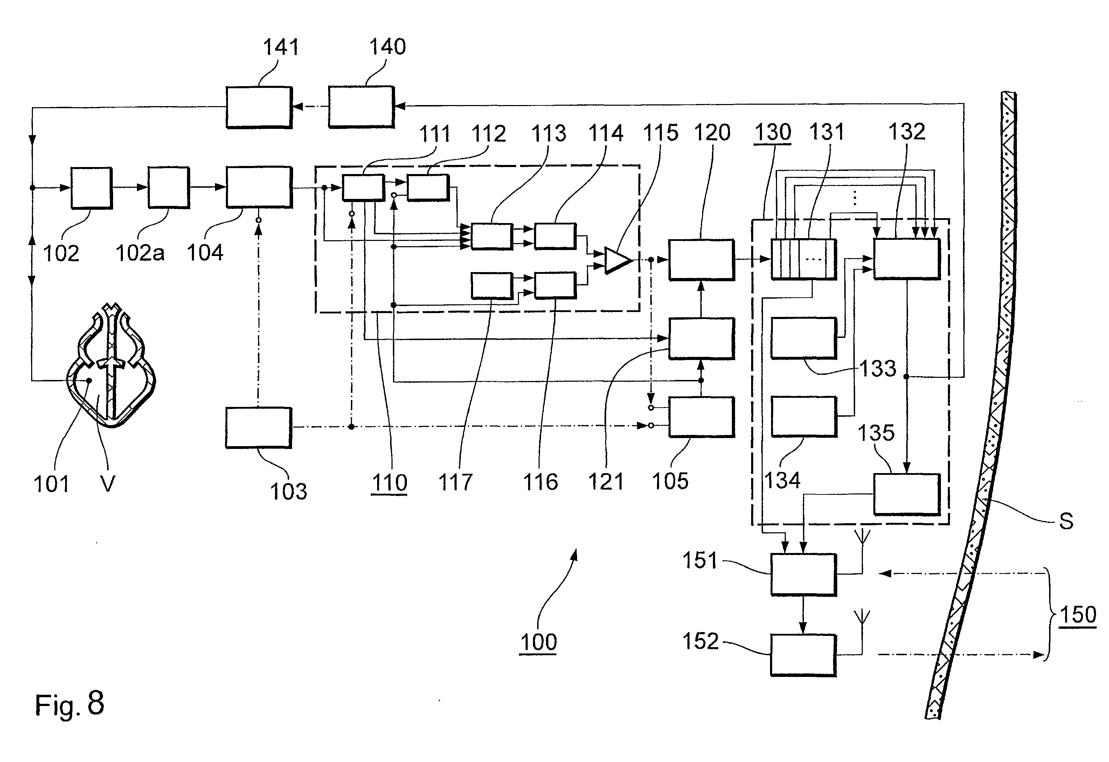

Fig. 8 zeigt ein stark vereinfachtes Funktions-Blockschaltbild eines Herzstimulators 100 mit einer Ausführungsform der erfindungsgemäßen Vorrichtung. (Bezüglich der konkreten Implementierung der letzteren wird auf Fig. 6 und 7 zurückverwiesen.)

Im Ventrikel V des Herzens eines Patienten ist eine der Aufnahme von Herzaktionspotentialen sowie der Zuführung von Stimulationsimpulsen zum Herzgewebe dienende Elektrode 101 angeordnet, die in üblicher Weise mit einem Abfühlverstärker 102 zur Verstärkung und Aufbereitung der Herzsignale verbunden ist. Dem Abfühlverstärker 102 ist ein A/D-Wandler 102a zur Digitalisierung der Amplitudenwerte nachgeordnet. Ein Taktgeber 103 steuert den Betrieb einer dem A/D-Wandler 102a nachgeordneten Sample&Hold-Schaltung 104 derart, daß in durch die Taktfrequenz vorgegebenen Zeitintervallen der Amplitudenwert des aufgenommenen Herzsignals registriert und jeweils bis zum nächsten Taktimpuls gehalten wird. Weiterhin ist der Taktgeber mit dem Eingang eines Zählers 105 sowie einem Steuereingang einer Signalkompressions- und berechnungseinheit 110 verbunden.

Die Einheit 110 umfaßt als Funktionseinheiten einen ersten Zwischenspeicher 111 für die vorletzte registrierte (im Sinne von Fig. 3 die "frühere") Signalprobe, in den auf das Taktsignal vom Taktgeber 103 hin der vorher in der Sample&Hold-Schaltung 104 gehaltene Signalwert (einschließlich Vorzeichen) umgeladen wird. Weiterhin umfaßt sie einen zweiten Zwischenspeicher 112, in dem die jeweils zuletzt im Zuge der Signalkompression verwendete Signalprobe (im Format Segmentlänge; Vorzeichen; Signalwert) gespeichert ist. Beide Speicher sowie der Ausgang der Sample&Hold-Schaltung 104 und der Zähler 105 sind mit Dateneingängen einer Arithmetikstufe 113 verbunden, die die - weiter oben genauer beschriebene - Berechnung des aktuellen Signalanstieges und des Anstieges zur letzten gespeicherten Signalprobe ausführt. Ausgangsseitig der Arithmetikstufe 113 stehen die Anstiegswerte bereit und werden den Eingängen einer Subtraktionsstufe 114 zugeführt, die die Differenz der Anstiege bestimmt.

Deren Ausgang ist mit einem Eingang eines Komparators 115 verbunden, dessen anderer Eingang mit dem Ausgang einer Multiplikationsstufe 116 verbunden ist. Diese wiederum ist eingangsseitig mit einem Schwellwertspeicher 117, in dem ein vorprogrammierter Anstiegs-Schwellwert (für die Segmentlänge 1) gespeichert ist, und dem Ausgang des Zählers 105 verbunden und berechnet den auf die aktuelle Segmentlänge bezogenen Anstiegs-Schwellwert, mit dem die festgestellte Anstiegsdifferenz zu vergleichen ist. Im Ergebnis des Vergleiches gibt der Komparator 115 (und damit die Berechnungseinheit 110) ein Steuersignal aus.

Dieses Steuersignal wird einerseits einem Rücksetzeingang des Zählers 105 zugeführt und setzt diesen immer dann zurück, wenn der Vergleich ergeben hat, daß die Anstiegsdifferenz größer als der aktuellen Schwellwert ist. Andererseits wird das Signal dem Eingang einer Speichersteuereinheit 120 zugeführt, deren Schaltsignal die Freigabe des Dateneingangs eines Signalformspeichers 131 bewirkt, in dessen Speicherbereichen jeweils eine komprimierte Signalform abgelegt wird.

Die diesem zugeführten Datenworte haben das Format (Segmentlänge; Vorzeichen; Amplitudenwert) und werden in einem Kodierer 121 aus den aktuellen Ausgangswerten des ersten Zwischenspeichers 111 und des Zählers 105 erzeugt, indem zugleich eine Amplitudenkodierung mit einer Zuordnunngstabelle in Art der in Fig. 4 gezeigten vorgenommen wird. Der bereichsweise separat und wahlfrei zugreifbare Signalformspeicher 131 ist mit Dateneingängen einer Signalform-Auswertungseinheit 132 verbunden. Diese prüft jeden aktuell ermittelten Gesamt-Signalverlauf eines intrakardial aufgenommenen EKG entsprechend in einem Betriebsprogrammspeicher 133 gespeicherten Programmen auf das Vorliegen vorgegebener (ebenfalls im Speicher 131 oder in einem separaten Datenspeicher 134 abgelegter) Signalformkriterien und/oder anhand von im Speicher 134 gespeicherten Signifikanzkriterien auf das Vorliegen signifikanter Abweichungen gegenüber früher ermittelten EKGs.

Im Speicher 134 sind zugleich Diagnosekriterien gespeichert, deren Verarbeitung mit den Signalform-Vergleichsdaten in einer hierarchisch übergeordneten zweiten Verarbeitungsebene in der Auswertungsstufe 132 es ermöglicht, in der Vorrichtung 100 autonom unmitelbar diagnostische Aussagen repräsentierende Daten zu gewinnen. Diese werden einerseits in einem Diagnosenspeicher 135 abgelegt und andererseits dem Eingang einer - als solche bekannten Stimulationssteuereinheit 140 zugeführt, die daraus die Steuersignale für einen Impulsgenerator 141 zur bedarfsweisen Stimulation des Ventrikels über die Elektrode 101 gewinnt und den Impulsgenerator entsprechend ansteuert - etwa im Falle der Feststellung eines Anfalles ventrikulärer Tachykardie anhand des Signalformvergleiches derart, daß eine die Tachykardie terminierende Impulsfolge abgegeben wird.

Sowohl die im Signalformspeicher 131 als auch die im Diagnosenspeicher 135 gespeicherten Daten stehen auf externen Abruf über eine Telemetrie-Strecke 150 zur Übertragung durch die Haut S nach außerhalb des Patienten bereit. Die (serielle) Übertragung der Daten zu einer Sendeeinheit 152 wird nach Empfang eines entsprechenden Abrufsignals durch eine Telemetrie-Steuereinheit 151 freigegeben und gesteuert. Die Vorrichtung 100 ist nach obigem als autarkes integriertes Diagnose- und Therapiegerät anzusprechen und steht wahlweise zusätzlich in Verbindung mit einem die Diagnoseergebnisse periodisch überwachenden Arzt. Zusätzlich können - in an sich bekannter Weise - Mittel zur internen, gegebenenfalls. auch zeitbezogenen, Registrierung von Stimulations- oder sonstigen Betriebsparametern vorgesehen sein, so daß zugleich eine retrospektive Betriebskontrolle des Gerätes möglich wird.

## Patentansprüche

1. Verfahren zur Speicherung und/oder Übertragung von einen zeitlichen Verlauf aufweisenden Signalen, bei dem der zeitliche Verlauf, insbesondere in vorgegebenen Zeitabständen mit konstantem Abtastintervall **(Δt),** erfasst und dadurch eine Menge von Signalproben **(A - X)** gewonnen wird, von denen im Ergebnis einer Selektion anhand eines vorbestimmten Auswahlkriteriums eine Teilmenge gespeichert und/oder übertragen wird, wobei das Auswahlkriterium der Wert der ersten Ableitung des zeitlichen Verlaufs der Signale nach der Zeit ist, und wobei die Selektion derart ausgeführt wird, dass der Wert der ersten Ableitung der geradlinigen Verbindung zwischen jeweils einer n-ten **(G)** und der (n-1)-ten Signalprobe **(F)** bestimmt und mit einem Referenzwert verglichen wird und die (n-1)-te Signalprobe **(F)** genau dann als neue letzte Signalprobe gespeichert und/oder übertragen wird, wenn sich der Wert der ersten Ableitungen vom Referenzwert um mehr als einen vorgegebenen Wert unterscheidet,
**dadurch gekennzeichnet, dass**
der Referenzwert durch die erste Ableitung der geradlinigen Verbindung zwischen der n-ten Signalprobe (G) und der letzten gespeicherten und/oder übertragenen Signalprobe (A) ermittelt wird
und das Verfahren derart ausgestaltet ist, dass es in einem implantierbaren medizinischen Gerät zur Speicherung und/oder Übertragung von einen zeitlichen Verlauf aufweisenden Signalen aus diesem nach außerhalb des Körpers des Patienten einsetzbar ist.

2. Verfahren nach Anspruch 1, **dadurch gekennzeichnet, daß** für jede gespeicherte und/oder übertragene Signalprobe das Vorzeichen, der Amplitudenwert und der Dauer des gegenüber der zuletzt gespeicherten Signalprobe verstrichenen Zeitintervalle, insbesondere in einem einzigen Datenwort, gespeichert bzw. übertragen werden.

3. Verfahren nach einem der vorangehenden Ansprüche, **dadurch gekennzeichnet, daß** im Falle der zeitnahen Übertragung der Signalproben der Teilfolge mindestens die jeweils zuletzt übertragene Signalprobe intern zwischengespeichert wird.

4. Verfahren nach einem der vorangehenden Ansprüche, **dadurch gekennzeichnet, daß** die Abtastung mit einer Digitalisierung der Amplitudenwerte verbunden ist.

5. Verfahren nach Anspruch 4, **dadurch gekennzeichnet, daß** die Digitalisierung kleiner Amplitudenwerte mit höherer Auflösung erfolgt als die größerer Amplitudenwerte.

6. Verfahren nach einem der vorangehenden Ansprüche, **dadurch gekennzeichnet, daß** die Signale Herzaktionssignale, insbesondere ein intrakardial aufgenommenes EKG, oder solche darstellende, von außerhalb des Körpers des Patienten übermittelte, Modell- bzw. Vergleichssignale sind.

7. Vorrichtung zur Durchführung des Verfahrens nach einem der vorangehenden Ansprüche, mit
- einem Zeitgeber **(103)** zur Festlegung von Abtastintervallen,
- einem Signalaufnehmer **(101, 102)** zur Aufnahme des Signal-Amplitudenwertes jeweils in den Abtastintervallen,
- einer mit dem Zeitgeber **(103)** und mindestens mittelbar mit dem Signalaufnehmer **(101, 102)** verbundenen Verarbeitungseinheit **(110)** zur Verarbeitung der aufgenommenen Signal-Amplitudenwerte entsprechend einem vorgegebenen Algorithmus und zur Ausgabe eines Speicher-Steuersignals im Ergebnis der Verarbeitung,
- einem mit dem Ausgang des Zeitgebers **(103)** und über einen Rücksetzeingang mit dem Steuersignalausgang der Verarbeitungseinheit **(110)** verbundenen Zähler **(105)** zur Zählung der Abtastintervalle im Ansprechen auf das Speicher-Steuersignal und zur Ausgabe eines Intervall-Zählwertes der auch der Verarbeitungseinheit **(110)** zugeführt wird,
- einem mindestens mittelbar mit dem Ausgang des Signalaufnehmers **(101, 102)** sowie dem Ausgang des Zählers **(105)** verbindbaren Speicher **(131)** zur Speicherung von Signalproben als Datenworte aus Zählerstand, Signal-Vorzeichen und Signalamplitude,
- wobei die Verarbeitungseinheit **(110)** aufweist:
- eine erste **(111)** und zweite **(112)** Zwischenspeichereinheit zur Zwischenspeicherung der vorletzten aufgenommenen Signalprobe sowie der zuletzt in den Speicher gespeicherten Sig-nalprobe,
- eine mindestens mittelbar mit den Ausgängen des Signalaufnehmers **(101, 102)** und des Zählers **(105)** sowie der ersten **(111)** und zweiten **(112)** Zwischenspeichereinheit verbundene Arithmetikstufe **(113)** zur Berechnung der ersten Ableitungen der geradlinigen Verbindung zwischen der aktuellen und der vorletzten Signalprobe sowie der geradlinigen Verbindung zwischen der **aktuellen** und der letzten gespeicherten Signalprobe,
- eine mit der Arithmetikstufe **(113)** verbundene Subtraktionsstufe **(114)** zur Bildung der Differenz aus den ersten Ableitungen,
- einen Schwellwertspeicher **(117)** zur Speicherung eines Differenzschwellwertes und
- eine eingangsseitig mit der Subtraktionsstufe **(114)** und dem Schwellwertspeicher **(117)** verbundene Vergleichereinheit **(115)** zum Vergleich der Differenz der ersten Ableitungen mit dem Differenz-Schwellwert und zur Ausgabe des Speicher-Steuersignals im Ergebnis des Vergleiches für die Speicherung der vorletzten Signalprobe.
- und einer mit dem **Steuersignalausgang der Verarbeitungseinheit (110)** verbundenen Speicherstevereinheit **(120)** zur Herstellung einer Verbindung zwischen dem **ersten Zwi- schenspeicher (111)** sowie dem Ausgang des Zählers **(105)** und dem Speicher **(131)** im Ansprechen auf das Speicher-Steuersignal und zum Bewirken einer Speicherung einer Signalprobe.

8. Vorrichtung zur Durchführung des Verfahrens nach einem der vorangehenden Ansprüche, mit
- einem Zeitgeber **(103)** zur Festlegung von Abtastintervallen,
- einem Signalaufnehmer **(101, 102)** zur Aufnahme des Signal-Amplitudenwertes jeweils in den Abtastintervallen,
- einer mit dem Zeitgeber **(103)** und mindestens mittelbar mit dem Signalaufnehmer **(101, 102)** verbundenen Verarbeitungseinheit **(110)** zur Verarbeitung der aufgenommenen Signal-Amplitudenwerte entsprechend einem vorgegebenen Algorithmus und zur Ausgabe eines Sende-Steuersignals im Ergebnis der Verarbeitung,
- einem mit dem Ausgang des Zeitgebers **(103)** und über einen Rücksetzeingang mit dem Steuersignalausgang der Verarbeitungseinheit **(110)** verbundenen Zähler **(105)** zur Zählung der Abtastintervalle im Ansprechen auf das Sende-Steuersignal und zur Ausgabe eines Intervall-Zählwertes, der auch der Verarbeitungseinheit **(110)** zugeführt wird,
- einem mindestens mittelbar mit dem Ausgang des Signalaufnehmers **(101, 102)** sowie dem Ausgang des Zählers **(105)** verbindbaren Sender **(152)** zur Übertragung von Signalproben als Datenworte aus Zählerstand, Signal-Vorzeichen und Signalamplitude nach außerhalb der Vorrichtung,
- wobei die Verarbeitungseinheit **(110)** aufweist:
- eine erste **(111)** und zweite **(112)** Zwischenspeichereinheit zur Zwischenspeicherung der vorletzten aufgenommenen Signalprobe sowie der zuletzt übertragenen Signalprobe,
- eine mindestens mittelbar mit den Ausgängen des Signalaufnehmers **(101, 102)** und des Zählers **(105)** sowie der ersten **(111)** und zweiten **(112)** Zwischenspeichereinheit verbundene Arithmetikstufe **(113)** zur Berechnung der ersten Ableitungen der geradlinigen Verbindung zwischen der aktuellen und der vorletzten Signalprobe sowie der geradlinigen Verbindung zwischen der **aktuellen** und der letzten **übertragenen** Signalprobe,
- eine mit der Arithmetikstufe **(113)** verbundene Subtraktionsstufe **(114)** zur Bildung der Differenz aus den ersten Ableitungen,
- einen Schwellwertspeicher **(117)** zur Speicherung eines Differenz-Schwellwertes und
- eine eingangsseitig mit der Subtraktionsstufe **(114)** und dem Schwellwertspeicher **(117)** verbundene Vergleichereinheit **(115)** zum Vergleich der Differenz der ersten Ableitungen mit dem Differenz-Schwellwert und zur Ausgabe des Sende-Steuersignals im Ergebnis des Vergleiches für die Übertragung der vorletzten Signalprobe.
- und einer mit dem **Steuersignalausgang der Verarbeitungseinheit** (110), verbundenen Senderstevereinheit (151) zur Herstellung einer Verbindung zwischen **dem ersten Zwischenspeicher (111)** sowie dem Ausgang des Zählers **(105)** und dem Sender **(152)** im Ansprechen auf das Sende-Steuersignal und zum Bewirken einer Übertragung einer Signalprobe

9. Vorrichtung nach Anspruch 7 **dadurch gekennzeichnet, daß** der Speicher **(131)** als Halbleiter-Direktzugriffsspeicher ausgebildet ist und eine Mehrzahl von getrennt zugreifbaren Speicherbereichen zur Speicherung jeweils einer Signalproben-Teilfolge aufweist.

10. Vorrichtung nach Anspruch 8, **dadurch gekennzeichnet, daß** die Sendeeinheit **(152)** als Telemetrie-Sendeeinheit ausgebildet ist.

11. Vorrichtung nach einem der Ansprüche 7 bis 10, **dadurch gekennzeichnet, daß** der Signalaufnehmer mindestens eine Elektrode **(101)** mit nachgeordnetem Abfühlverstärker **(102)** zur Erfassung von Herzaktionspotentialen aufweist.

12. Vorrichtung nach einem der Ansprüche 7 bis 11, **dadurch gekennzeichnet, daß** sowohl ein Speicher **(131)** als auch eine Sendeeinheit **(152)** vorgesehen ist, wobei die Sendeeinheit **(152)** mit dem Speicher **(131)** zur Übertragung von Speicherinhalten nach außerhalb des Gerätes verbindbar ist und Mittel zur Aktivierung der Sendeeinheit **(152)** auf Abruf oder in vorgegebenen Zeitabständen vorgesehen sind.

13. Vorrichtung nach einem der Ansprüche 7 bis 12, **dadurch gekennzeichnet, daß** eine Steuer- oder Diagnosedaten-Berechnungseinheit **(132)** vorgesehen ist, die eingangsseitig mit dem Speicher **(131)** für die Signalproben-Teilfolgen und ausgangsseitig wahlweise mit **einem Steuer- oder Diagnosedatenspeicher (135)** verbunden und zur vergleichenden Verarbeitung verschiedener gespeicherter Signalproben zur Gewinnung von Steuersignalen oder diagnostisch relevanten Daten ausgebildet ist.

14. **Implantierbares Antiarrhythmiegerät umfassend eine Vorrichtung nach einem der Ansprüche 7 bis 13.**

15. Implantierbares Antiarrhythmiegerät umfassend eine Vorrichtung nach Anspruch 13, **dadurch gekennzeichnet, daß** eine eingangsseitig mit dem Ausgang der Steuerdaten-Berechnungseinheit **(132)** und/oder dem Steuerdatenspeicher **(135)** in Verbindung stehende Betriebssteuereinheit **(140)** vorgesehen ist, welche zur Steuerung des Betriebs des Antiarrhythmiegerätes aufgrund des Ergebnisses der vergleichenden Auswertung ausgebildet ist.

## Claims

1. A method for storing and/or transmitting signals having a temporal course, in which the temporal course is recorded, in particular in predetermined time intervals with a constant sampling interval (Δt), and consequently a quantity of signal samples (A - X) is obtained, of which as a result of a selection using a predetermined selection criterion a sub-set is stored and/or transmitted, whereby the selection criterion is the value of the first derivative of the temporal course of the signals according to time, and whereby the selection is performed in such a manner that the value of the first derivative of the linear connection between in each case an n^{th} (G) and the (n-1)^{th} signal sample (F) is determined and compared with a reference value and the (n-1)^{th} signal sample (F) is then precisely stored as a new last signal sample and/or is transmitted when the value of the first derivatives differs from the reference value by more than a predetermined value,
**characterised in that**
the reference value is determined by the first derivative of the linear connection between the n^{th} signal sample (G) and the signal sample (A) which was the last to be stored and/or transmitted
and the method is configured in such a manner that it can be used in an implantable medical apparatus for storing and/or transmitting signals having a temporal course from said apparatus to outside the patient's body.

2. A method according to Claim 1,
**characterised in that** for each stored and/or transmitted signal sample the sign, the amplitude value and the length of time intervals that have elapsed with respect to the last signal sample stored, are stored or transmitted, in particular in a single data word.

3. A method according to one of the preceding Claims,
**characterised in that** in the event of the real-time transmission of signal samples of the sub-sequence, at least the signal sample which was last transmitted in each case is internally intermediately stored.

4. A method according to one of the preceding Claims,
**characterised in that** the sampling is connected to a digitalisation of the amplitude values.

5. A method according to Claim 4,
**characterised in that** the digitalisation of small amplitude values takes place with higher resolution than the larger amplitude values.

6. A method according to one of the preceding Clams,
**characterised in that** the signals are cardiac action signals, in particular an intracardially recorded ECG, or model or comparison signals which represent them and are transmitted from outside the patient's body.

7. A device for performing the method according to one of the preceding Claims, having
- a timing circuit (103) for determining sampling intervals,
- a signal transducer (101, 102) for recording the signal amplitude value in the sampling intervals in each case,
- a processing unit (110), which is connected to the timing circuit (103) and at least indirectly to the signal transducer (101, 102), for processing the recorded signal amplitude values corresponding to a predetermined algorithm and for outputting a memory control signal as a result of the processing,
- a counter (105), which is connected to the output of the timing circuit (103) and via a reset input to the control signal output of the processing unit (110), for counting the sampling intervals in response to the memory control signal and for outputting an interval count value which is also supplied to the processing unit (110),
- a memory (131), which can be connected at least indirectly to the output of the signal transducer (101, 102) and also the output of the counter (105), for storing signal samples as data words from counter reading, signal sign and signal amplitude,
- wherein the processing unit (110) comprises:
- a first (111) and a second (112) intermediate memory unit for the intermediate storage of the penultimately recorded signal sample and also of the signal sample that was last stored in the memory,
- an arithmetic stage (113), which is connected at least indirectly to the outputs of the signal transducer (101, 102) and of the counter (105) and also of the first (111) and second (112) intermediate memory unit, for calculating the first derivatives of the linear connection between the present and the penultimate signal sample and also the linear connection between the current and the last stored signal sample,
- a subtraction stage (114) connected to the arithmetic stage (113) for forming the difference from the first derivatives,
- a threshold value memory (117) for storing a difference threshold value and
- a comparator unit (115), which is connected on the input side to the subtraction stage (114) and the threshold value memory (117), for comparing the difference of the first derivatives with the difference threshold value and for outputting the memory control signal as a result of the comparison for the storage of the penultimate signal sample.
- and a memory control unit (120) connected to the control signal output of the processing unit (110) for producing a connection between the first intermediate memory (111) and also the output of the counter (105) and the memory (131) in response to the memory control signal and to effect a storage of a signal sample.

8. A device for performing the method according to one of the preceding Claims, having
- a timing circuit (103) for determining sampling intervals,
- a signal transducer (101, 102) for recording the signal amplitude value in the sampling intervals in each case,
- a processing unit (110), which is connected to the timing circuit (103) and at least indirectly to the signal transducer (101, 102), for processing the recorded signal amplitude values corresponding to a predetermined algorithm and for outputting a transmitted control signal as a result of the processing,
- a counter (105), which is connected to the output of the timing circuit (103) and via a reset input to the control signal output of the processing unit (110), for counting the sampling intervals in response to the transmit control signal and for outputting an interval count value which is also supplied to the processing unit (110),
- a transmitter (152), which can be connected at least indirectly to the output of the signal transducer (101, 102) and also to the output of the counter (105), for transmitting signal samples as data words from counter reading, signal sign and signal amplitude to outside the device,
- wherein the processing unit (110) comprises:
- a first (111) and a second (112) intermediate memory unit for the intermediate storage of the penultimately recorded signal sample and also the signal sample which was the last transmitted,
- an arithmetic stage (113), which is connected at least indirectly to the outputs of the signal transducer (101, 102) and of the counter (105) and also the first (111) and second (112) intermediate storage unit, for calculating the first derivatives of the linear connection between the current and the penultimate signal sample and also of the linear connection between the current and the last transmitted signal sample,
- a subtraction stage (114) connected to the arithmetic stage (113) for forming the difference from the first derivatives,
- a threshold value memory (117) for storing a difference threshold value and
- a comparator unit (115), which is connected at the input side to the subtraction stage (114) and the threshold value memory (117), for comparing the difference of the first derivatives with the difference threshold value and for outputting the transmit control signal as a result of the comparison for the transmission of the penultimate signal sample.
- and a transmit control unit (151) connected to the control signal output of the processing unit (110) for producing a connection between the first intermediate memory (111) and also the output of the counter (105) and the transmitter (152) in response to the transmit control signal and for effecting a transmission of a signal sample.

9. A device according to Claim 7,
**characterised in that** the memory (131) is constructed as a semiconductor random-access memory and comprises a plurality of separately accessible memory areas for storing a signal sample sub-sequence in each case.

10. A device according to Claim 8,
**characterised in that** the transmit unit (152) is constructed as a telemetry transmit unit.

11. A device according to one of Claims 7 to 10,
**characterised in that** the signal transducer comprises at least one electrode (101) with a scanning amplifier (102) connected after it for recording cardiac action potentials.

12. A device according to one of Claims 7 to 11,
**characterised in that** both a memory (131) and a transmit unit (152) is provided, with it being possible to connect the transmit unit (152) to the memory (131) for the transmission of storage contents to outside the apparatus and means are provided for activating the transmit unit (152) when polled or in predetermined time intervals.

13. A device according to one of Claims 7 to 12,
**characterised in that** a control or diagnosis data computation unit (132) is provided, which is connected at the input side to the memory (131) for the signal sample sub-sequences and at the output side alternatively with a control or diagnosis data memory (135) and is constructed for the comparative processing of various stored signal samples for obtaining control signals or diagnostically relevant data.

14. An implantable anti-arrhythmia apparatus comprising a device according to one of Claims 7 to 13.

15. An implantable anti-arrhythmia apparatus, comprising a device according to Claim 13,
**characterised in that** an operating control unit (140) connected at the input side to the output of the control data computation unit (132) and/or the control data memory (135) is provided, which is constructed to control the operation of the anti-arrhythmia apparatus on the basis of the result of the comparative evaluation.

## Revendications

1. Procédé pour mémoriser et/ou transmettre des signaux présentant une variation dans le temps, selon lequel on détecte la variation dans le temps notamment pendant des intervalles de temps prédéterminés avec un intervalle d'échantillonnage constant (Δt), et on détermine de ce fait une quantité d'échantillons de signaux (A-X), dont une quantité partielle est mémorisée et/ou transmise en tant que résultat d'une sélection sur la base d'un critère de sélection prédéterminé, et selon lequel le critère de sélection est la valeur de dérivée première de la variation dans le temps des signaux, et selon lequel la sélection est réalisée de telle sorte qu'on détermine la valeur de la dérivée première de la liaison rectiligne entre respectivement un n-ème échantillon de signal (G) et le (n-1)-ème échantillon de signal (F) et on la compare à une valeur de référence, et on mémorise et/ou on transmet le (n-1)-ème échantillon de signal (F) de façon précise en tant que nouveau dernier échantillon de signal lorsque la valeur de la dérivée première diffère de la valeur de référence de plus d'une valeur prédéterminée, **caractérisé en ce que**
on détermine la valeur de référence au moyen de la dérivée première de la liaison rectiligne entre le n-ème échantillon de signal (G) et le dernier échantillon de signal (A) mémorisé et/ou transmis, et
le procédé est agencé de telle sorte qu'il peut être utilisé dans un appareil médical implantable pour mémoriser et/ou transmettre des signaux présentant une variation dans le temps, à partir de cet appareil vers l'extérieur du corps du patient.

2. Procédé selon la revendication 1, **caractérisé en ce que** pour chaque échantillon de signal mémorisé et/ou transmis, le signe, la valeur d'amplitude et la durée de l'intervalle qui s'est écoulé depuis l'échantillon de signal mémorisé en dernier lieu, sont mémorisés ou transmis notamment dans un seul mot de données.

3. Procédé selon l'une des revendications précédentes, **caractérisé en ce que** dans le cas de la transmission, rapprochée dans le temps, des échantillons de signaux de la suite partielle, au moins l'échantillon de signal respectivement transmis en dernier lieu est mémorisé temporairement de façon interne.

4. Procédé selon l'une des revendications précédentes, **caractérisé en ce que** l'échantillonnage est lié à une numérisation des valeurs d'amplitude.

5. Procédé selon la revendication 4, **caractérisé en ce que** la numérisation de faible valeur d'amplitude s'effectue avec une résolution plus élevée que pour les valeurs d'amplitude plus élevées.

6. Procédé selon l'une des revendications précédentes, **caractérisé en ce que** les signaux d'activité cardiaque, notamment un électrocardiogramme enregistré d'une manière intracardiaque ou des signaux de modèles ou de comparaison, qui représentent de tels signaux d'activité cardiaque et sont transmis à partir de l'extérieur du corps du patient.

7. Dispositif pour la mise en oeuvre du procédé selon l'une des revendications précédentes, comportant:
- une minuterie (130) pour déterminer des intervalles d'échantillonnage,
- un récepteur de signaux (101, 102) pour la réception de la valeur d'amplitude du signal respectivement dans les intervalles d'échantillonnage,
- une unité de traitement (110) qui est reliée à la minuterie (130) et au moins indirectement au récepteur de signaux (101, 102) pour traiter les valeurs d'amplitude de signaux reçus, conformément à un algorithme prédéterminé et pour la délivrance d'un signal de commande de mémoire en tant que résultat du traitement,
- un compteur (105) qui est relié à la sortie de la minuterie (130) est relié par l'intermédiaire d'une entrée de remise à zéro à la sortie du signal de commande de l'unité de traitement (110), pour le comptage des intervalles d'échantillonnage en réponse au signal de commande de mémoire et pour la délivrance d'une valeur de comptage d'intervalles, qui est également envoyée à l'unité de traitement (110),
- une mémoire (131), qui peut être reliée au moins indirectement à la sortie du récepteur de signaux (101, 102) ainsi qu'à la sortie du compteur (105), pour la mémorisation d'échantillons de signaux en tant que mots de données à partir de l'état du compteur, du signe du signal et de l'amplitude du signal,
- l'unité de traitement (110) comportant:
- une première unité (111) et une seconde unité (112) de mémoire intermédiaire pour réaliser la mémorisation temporaire de l'avant-dernier échantillon de signal reçu ainsi que de l'échantillon de signal mémorisé en dernier dans la mémoire,
- un étage arithmétique (113), qui est relié au moins indirectement aux sorties du récepteur de signaux (101, 102) et du compteur (105) ainsi que la première unité de mémoire intermédiaire (111) et de la seconde unité de mémoire intermédiaire (112), pour le calcul des dérivées premières de la liaison rectiligne entre l'échantillon de signal actuel et l'avant-dernier échantillon de signal ainsi que de la liaison rectiligne entre l'échantillon de signal actuel et le dernier échantillon de signal mémorisé,
- un étage de soustraction (114) qui est relié à l'étage arithmétique (113) et sert à former la différence à partir des dérivées premières,
- une mémoire à valeur de seuil (117) pour mémoriser une valeur de seuil de différence, et
- une unité formant comparateur (115) qui est reliée côté entrée à l'étage de soustraction (114) et à la mémoire à valeur de seuil (117), pour comparer la différence des dérivées premières à la valeur de seuil de différence et pour délivrer le signal de commande de mémoire en tant que résultat de la comparaison pour la mémorisation de l'avant-dernier échantillon de signal, et
- une unité de commande de mémoire (120), qui est reliée à la sortie du signal de commande de l'unité de traitement (110), pour l'établissement d'une liaison entre la première mémoire intermédiaire (111) ainsi que la sortie du compteur (105) et la mémoire (131) en réponse au signal de commande de mémoire et pour réaliser une mémorisation d'un échantillon de signal.

8. Dispositif pour la mise en oeuvre du procédé selon l'une des revendications précédentes, comportant
- une minuterie (130) pour déterminer des intervalles d'échantillonnage,
- un récepteur de signaux (101, 102) pour la réception de la valeur d'amplitude du signal respectivement dans les intervalles d'échantillonnage,
- une unité de traitement (110) qui est reliée à la minuterie (130) et au moins indirectement au récepteur de signaux (101, 102) pour traiter les valeurs d'amplitude de signaux reçus, conformément à un algorithme prédéterminé et pour la délivrance d'un signal de commande de mémoire en tant que résultat du traitement,
- un compteur (105) qui est relié à la sortie de la minuterie (130) est relié par l'intermédiaire d'une entrée de remise à zéro à la sortie du signal de commande de l'unité de traitement (110), pour le comptage des intervalles d'échantillonnage en réponse au signal de commande de mémoire et pour la délivrance d'une valeur de comptage d'intervalles, qui est également envoyée à l'unité de traitement (110),
- une mémoire (131), qui peut être reliée au moins indirectement à la sortie du récepteur de signaux (101, 102) ainsi qu'à la sortie du compteur (105), pour la mémorisation d'échantillons de signaux en tant que mots de données à partir de l'état du compteur, du signe du signal et de l'amplitude du signal,
- un émetteur (152), qui peut être relié au moins directement à la sortie du récepteur de signaux (101, 102) ainsi qu'à la sortie du compteur (105), pour la transmission d'échantillons de signaux en tant que mots de données à partir d'un étage du compteur, d'un signe du signal et d'une amplitude du signal, à l'extérieur du dispositif,
- l'unité de traitement (110) comportant:
- une première unité (111) et une seconde unité (112) de mémoire intermédiaire pour réaliser la mémorisation temporaire de l'échantillon de signal reçu en avant de l'avant-dernier échantillon de signal reçu ainsi que de l'échantillon de signal mémorisé en dernier dans la mémoire,
- un étage arithmétique (113), qui est relié au moins indirectement aux sorties du récepteur de signaux (101, 102) et du compteur (105) ainsi que la première unité de mémoire intermédiaire (111) et de la seconde unité de mémoire intermédiaire (112), pour le calcul des dérivés premières de la liaison rectiligne entre l'échantillon de signal actuel et l'avant-dernier échantillon de signal ainsi que de la liaison rectiligne entre l'échantillon de signal actuel et le dernier échantillon de signal mémorisé,
- un étage de soustraction (114) qui est relié à l'étage arithmétique (113) et sert à former la différence à partir des dérivées premières,
- une mémoire à valeur de seuil (117) pour mémoriser une valeur de seuil de différence, et
- une unité formant comparateur (104) qui est reliée côté entrée à l'étage de soustraction (114) et à la mémoire à valeur de seuil (117), pour comparer la différence des dérivées premières à la valeur de seuil de différence et pour délivrer le signal de commande de mémoire en tant que résultat de la comparaison pour la mémorisation de l'avant-dernier échantillon de signal, et
- une unité de commande d'émetteur (151), qui est reliée à la sortie du signal de commande de l'unité de traitement (110), pour l'établissement d'une liaison entre la première mémoire intermédiaire (111) ainsi que la sortie du compteur (105) et l'émetteur (152) en réponse au signal de commande d'émetteur et pour réaliser une émission d'un échantillon de signal.

9. Dispositif selon la revendication 7, **caractérisé en ce que** la mémoire (131) est agencée sous la forme d'une mémoire à accès direct à semiconducteurs et comporte une multiplicité de zones de mémoire auxquelles l'accès peut être réalisé séparément et qui sont utilisées pour la mémorisation respectivement d'une suite partielle d'échantillons de signaux.

10. Dispositif selon la revendication 8, **caractérisé en ce que** l'unité d'émission (152) est agencée sous la forme d'une unité d'émission de télémétrie.

11. Dispositif selon l'une des revendications 7 à 10, **caractérisé en ce que** le récepteur de signaux comporte au moins une électrode (101) possédant des amplificateurs de détection (102) branchés en aval et servant à détecter des potentiels d'activité cardiaque.

12. Dispositif selon l'une des revendications 7 à 11, **caractérisé en ce qu'**il est prévu aussi bien une mémoire (131) qu'une unité d'émission (152), l'unité d'émission (152) pouvant être reliée à la mémoire (131) par la transmission de contenu de la mémoire vers l'extérieur de l'appareil, et des moyens pour activer l'unité d'émission (152) lors d'un appel ou pendant des intervalles de temps prédéterminés.

13. Dispositif selon l'une des revendications 7 à 12, **caractérisé en ce qu'**il est prévu une unité (132) de calcul de données de commande et de diagnostic, qui est reliée côté entrée à la mémoire (131) pour les suites partielles d'échantillons de signaux et est reliée côté sortie au choix à une mémoire de données de commande ou de données de diagnostic (135) et est agencée pour le traitement comparatif de différents échantillons de signaux mémorisés, pour l'obtention de signaux de données ou de données importantes du point de vue diagnostic.

14. Dispositif anti-arythmie implantable comprenant un dispositif selon l'une des revendications 7 à 13.

15. Appareil anti-arythmie implantable comprenant un dispositif selon la revendication 13, **caractérisé en ce qu'**il est prévu une unité de commande de fonctionnement (140), qui est reliée côté entrée à la sortie de l'unité de calcul de données de commande (132) et/ou à la mémoire de données de commande (135) et qui est agencée pour réaliser la commande du fonctionnement de l'appareil anti-arythmies sur la base du résultat de l'évaluation comparative.
